# EUROPÄISCHE PATENTSCHRIFT

(11) **EP 3 151 879 B1**
(45) Veröffentlichungstag und Bekanntmachung des Hinweises auf die Patenterteilung: **04.07.2018**
(21) Anmeldenummer: 15728407.6
(22) Anmeldetag: 28.05.2015
(51) Int. Cl.: A61M 1/28, A61M 1/14, A61M 1/34, A61B 5/021

(54) **DIALYSEGERÄT**
DIALYSIS APPARATUS
APPAREIL DE DIALYSE

(30) Priorität: 04.06.2014 DE 102014008367
(43) Veröffentlichungstag der Anmeldung: 12.04.2017
(73) Patentinhaber: Fresenius Medical Care Deutschland GmbH, 61352 Bad Homburg (DE)
(72) Erfinder: HÖRNIG, Norbert, 97509 Kolitzheim (DE)
(74) Vertreter: Herrmann, Uwe
(86) Internationale Anmeldenummer: PCT/EP2015/001093
(87) Internationale Veröffentlichungsnummer: WO 2015/185197

(56) Entgegenhaltungen:
- WO-A1-2013/051927
- US-A1- 2002 193 691
- US-A1- 2005 234 384

## Beschreibung

Die vorliegende Erfindung betrifft ein Dialysegerät, insbesondere ein Peritonealdialysegerät, mit wenigstens einem Kompressor und mit wenigstens einem pneumatischen Verbraucher, der mit dem Kompressor derart in Verbindung steht, dass dieser von dem Kompressor mit Druckluft beaufschlagbar ist.

Aus dem Stand der Technik sind Peritonealdialysegeräte bekannt, die über einen Kompressor verfügen, der bei Bedarf eine Mehrzahl von Verbrauchern mit Druckluft versorgt. Dazu gehören beispielsweise die pneumatischen Aktuatoren in Form von Pneumatikventilen, die die Flusswege in dem Disposable steuern, das während der Behandlung des Patienten mit dem Peritonealdialysegerät gekoppelt ist. Weitere Beispiele für pneumatische Aktuatoren, die von dem Kompressor mit Druckluft versorgt werden können, sind Entlüftungsventile an der Hydraulikpumpe, die für das Entgasen der Hydraulikflüssigkeit benötigt werden, sowie ein Luftkissen unterhalb der Schublade, das zum Verpressen des Disposables zwischen der Schublade und dem Maschinenblock dient. Der Pneumatikdruck ist üblicherweise auf einen bestimmten Druckwert, z.B. 2 bar begrenzt.

Die Steuerung des Kompressors erfolgt über einen Aktionsrechner.

Aus der US 2003/0120170 A1 ist eine Vorrichtung zur Bestimmung des Körperwassers bekannt, die auf einer Widerstandsmessung basiert. Während dieser Körperwasserbestimmung wird der Blutdruck in einem bestimmten Bereich gehalten. Die US 2001/0012917 A1 sowie die US 2002/0193691 A1 beschreiben die Blutdruckmessung eines Patienten während einer Dialysebehandlung. Weitere relevante Stand der Technik ist bekannt aus den Dokumenten US2005/0234384 A1 und WO2013/051927 A1. Der vorliegenden Erfindung liegt die Aufgabe zugrunde, ein Dialysegerät, insbesondere ein Peritonealdialysegerät dahingehend weiterzubilden, dass die Durchführung der Dialysebehandlung vereinfacht wird.

Diese Aufgabe wird durch ein Dialysegerät mit den Merkmalen des Anspruchs 1 gelöst. Danach ist vorgesehen, dass das Dialysegerät mit wenigstens einer Blutdruckmanschette derart in Verbindung steht oder verbindbar ist, dass die Blutdruckmanschette durch den Kompressor des Dialysegerätes mit Druckluft versorgbar ist. Der vorliegenden Erfindung liegt somit der Gedanke zugrunde, ein Dialysegerät der eingangs genannten Art dahingehend weiterzubilden, dass der Kompressor des Gerätes nicht nur zur Versorgung von herkömmlichen pneumatischen Aktuatoren, sondern auch zur Druckluftversorgung einer Blutdruckmanschette dient. Dies hat den Vorteil, dass sich die Blutdruckmessung direkt in das Dialysegerät integrieren lässt und vorzugsweise kein weiteres separates Gerät benötigt wird, das zusätzlich vom Patienten bzw. vom Nutzer zu bedienen ist.

Der Begriff "Kompressor" ist allgemein zu verstehen und umfasst nicht nur einen Verdichter im engeren Sinne, sondern jede beliebige Druckluftquelle.

In einer bevorzugten Ausgestaltung der Erfindung sind sämtliche zur Blutdruckmessung und ggf. Verarbeitung der gemessenen Daten erforderlichen Komponenten mit Ausnahme der Blutdruckmanschette in das Dialysegerät integriert.

Gemäß der Erfindung dient der Kompressor des Dialysegerätes somit nicht nur zur Versorgung der aus dem Stand der Technik bekannten Verbraucher, wie Ventilen oder dem Luftkissen zur Fixierung der Disposable-Kassette, sondern auch zur Druckluftversorgung der Blutdruckmanschette. Ein separater, eigens zur Druckluftversorung der Blutdruckmanschette vorhandener Kompressor, Luftpumpe etc. wird erfindungsgemäß somit nicht benötigt.

Vorzugsweise ist vorgesehen, dass das Dialysegerät wenigstens einen Anschluss aufweist, mit dem die Blutdruckmanschette lösbar verbunden oder lösbar verbindbar ist. Bei diesem Anschluss kann es sich um eine Steckverbindung handeln. Dies erlaubt einen besonders einfachen Anschluss der Blutdruckmanschette an das Dialysegerät. Gemäß der Erfindung ist vorgesehen, dass sich zwischen dem Kompressor und der Blutdruckmanschette wenigstens ein Ventil befindet, mittels dessen eine oder mehrere Leitungen, insbesondere die Druckluftleitung zwischen dem Kompressor und der Blutdruckmanschette absperrbar oder freigebbar sind.

Wird keine Blutdruckmessung benötigt, steht das Ventil vorzugsweise auf der Schließstellung, d.h. die Druckleitung zwischen der Druckseite des Kompressors und der Blutdruckmanschette ist gesperrt. Soll der Blutdruck gemessen werden, wird das Ventil geöffnet, so dass die Blutdruckmanschette von dem Kompressor mit Druckluft versorgt werden kann. Das Ventil ist als Mehrwegeventil und vorzugsweise als 2-Wege-Ventil ausgebildet, das derart ausgeführt ist, dass entweder die Blutdruckmanschette oder einer oder mehrere der sonstigen Verbraucher mit dem Kompressor in Druckluft-Verbindung stehen.

In einer weiteren Ausgestaltung der Erfindung weist das Dialysegerät eine Steuerungs- oder Regelungseinheit aufweist, die derart ausgebildet ist, dass diese die Versorgung der Blutdruckmanschette mit Druckluft und/oder den Vorgang der Blutdruckmessung steuert oder regelt. Diese Steuerungs- oder Regelungseinheit kann derart ausgebildet sein, dass sie das genannte Ventil öffnet und den Kompressor in Betrieb setzt, um die Blutdruckmanschette mit Druckluft zu versorgen. Die genannte Einheit kann des Weiteren den gesamten Ablauf der Blutdruckmessung einschließlich der Druckluftbeaufschlagung der Blutdruckmanschette steuern oder regeln. Auch die Verarbeitung und/oder Speicherung und/oder Anzeige der gemessenen Daten kann durch die Steuerungs- oder Regelungseinheit vollzogen werden.

Bei der genannten Steuerungs- oder Regelungseinheit handelt es sich vorzugsweise um die Einheit, die auch den Betrieb des Dialysegerätes steuert oder regelt. Vorzugsweise ist somit vorgesehen, dass die Blutdruckmessung über die Software des Dialysegerätes erfolgt.

In einer weiteren Ausgestaltung der Erfindung ist vorgesehen, dass wenigstens ein Drucksensor zur Erfassung des Blutdruckes vorhanden ist, wobei dieser Sensor einen Bestandteil des Dialysegerätes bildet oder mit dem Dialysegerät in Verbindung steht. Vorzugsweise ist der gemessene Druckwert an dem Dialysegerät anzeigbar. Vorzugsweise kann als Drucksensor ein bereits in dem Dialysegerät vorhandener Drucksensor verwendet werden, so dass kein eigens für die Blutdruckmessung vorzusehender Drucksensor notwendig ist.

Die Aufnahme des Druckes in der Blutdruckmanschette kann somit über einen Drucksensor im Dialysegerät erfolgen, wobei vorzugsweise vorgesehen ist, dass dazu ein bereits in dem Gerät vorhandener und auch für andere Druckmessungen benötigter Drucksensor verwendet wird.

Vorzugsweise ist vorgesehen, dass der gemessene Blutdruckwert an dem Display des Dialysegerätes angezeigt wird. Auch hierbei gilt, dass vorzugsweise ein bereits an dem Gerät vorhandenes Display zur Anzeige des oder der Blutdruckwerte verwendet wird.

In einer bevorzugten Ausgestaltung der Erfindung ist vorgesehen, dass das Dialysegerät derart ausgebildet ist, dass die Datenerfassung, Speicherung und Verarbeitung der mittels der Blutdruckmanschette gemessen Daten in dem Dialysegerät erfolgt. Dementsprechend können in dem Dialysegerät Prozessoren, Speicher etc. vorhanden sein, die die seitens des Drucksensors erfassten Daten verarbeiten und/oder speichern.

In einer weiteren bevorzugten Ausgestaltung der Erfindung ist vorgesehen, dass sämtliche zur Blutdruckmessung und Verarbeitung der gemessenen Daten erforderlichen Mittel außer der Blutdruckmanschette in dem Dialysegerät angeordnet sind. In dieser Ausführungsform der Erfindung muss ausschließlich eine Blutdruckmanschette vorhanden sein bzw. an das Dialysegerät angesteckt werden. Sämtliche weitere Komponenten, die im Rahmen der Blutdruckmessung erforderlich sind, wie beispielsweise der oder die Drucksensoren, Kompressor, Steuerungs- oder Regelungseinheiten, Anzeige, Speicher, Verarbeitungseinheiten zur Verarbeitung der Daten etc. sind vorzugsweise Bestandteile des Dialysegerätes. Dies gilt entsprechend auch für Betätigungselemente, wie Tasten, Schalter oder dergleichen, mittels derer die Blutdruckmessung nutzerseitig initiiert werden kann.

Grundsätzlich ist von der Erfindung auch umfasst, dass die Blutdruckmessung seitens des Dialysegerätes vollautomatisch abläuft, beispielsweise in bestimmten vorgegebenen Zeitabständen.

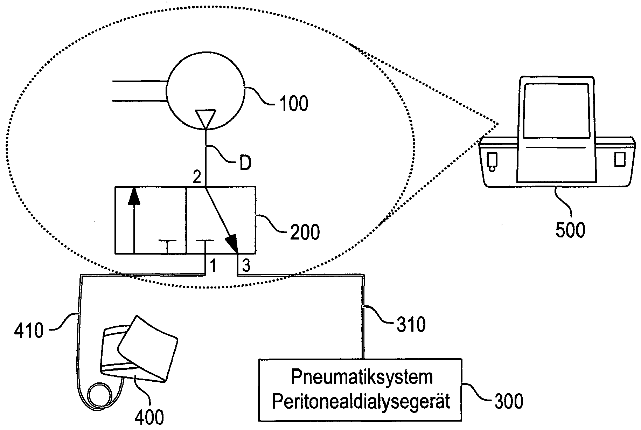

Weitere Vorteile und Einzelheiten der Erfindung werden anhand eines in der Zeichnung dargestellten Ausführungsbeispiels näher erläutert.

Die einzige Figur zeigt eine schematische Darstellung des Kompressors mit Ventil und Blutdruckmanschette und Pneumatiksystem des Peritonealdialysegerätes.

Das Bezugszeichen 100 kennzeichnet einen Kompressor eines Peritoneldialysegerätes.

Die Druckseite D des Kompressors 100 steht je nach Stellung des 3/2-WegeVentils 200 wahlweise mit dem Pneumatiksystem 300 des Peritonealdialysegerätes in Verbindung oder mit der Blutdruckmanschette 400.

Das Pneumatiksystem 300 des Peritonealdialysegerätes umfasst beispielsweise Pneumatikventile zum Schalten der Flüssigkeitswege in einer Disposablekassette, zum Schalten der Entlüftungsventile an der Hydraulikpumpe des Peritonealdialysegerätes, die für das Entgasen der Hydraulikflüssigkeit benötigt werden sowie zur Druckluftbeaufschlagung eines Luftkissens zum Verpressen des Disposablesets zwischen der Schublade und dem Maschinenblock des Peritonealdialysegerätes.

Vorzugsweise ist eine Druckbegrenzungseinrichtung vorgesehen, die den Druck auf der Druckseite D des Kompressors 100 auf einen Maximalwert, wie beispielsweise 2 bar begrenzt.

Das Bezugszeichen 500 kennzeichnet das Gehäuse, in dem sich der Kompressor 100 sowie das Ventil 200 befinden.

Das Peritonealdialysegerät verfügt über eine Software bzw. über eine Steuerungs- oder Regelungseinheit, die nicht nur den Betrieb des Peritonealdialysegerätes, sondern auch die Blutdruckmessung durchführt. Denkbar ist es auch, dass die Steuerung oder Regelung für die Blutdruckmessung von der des Peritonealdialysegerätes getrennt ist.

Soll eine Blutdruckmessung durchgeführt werden, was durch das Betätigen einer Taste oder dergleichen oder automatisiert durch das Gerät erfolgen kann, wird das Ventil 200 durch einen Aktuator etc. von seiner in der Figur dargestellten Position derart bewegt, so dass die Druckseite des Kompressors 100 mit der Blutdruckmanschette 400 über die Leitung 410 verbunden ist und Druckluft in die Blutdruckmanschette 400 eingeführt werden kann. In dieser Ventilstellung sind die Anschlüsse 2 und 1, nicht jedoch 2 und 3 verbunden; somit besteht keine Verbindung zwischen der Druckseite des Kompressors 100 und dem Pneumatiksystem 300 des Peritonealdialysegerätes.

Nach der Beendigung der Blutdruckmessung kann die Luft über dieselbe Leitung 410 wieder abgelassen werden.

Der Ablauf der Blutdruckmessung wird vorzugsweise durch eine Steuerungs- oder Regelungseinheit durchgeführt. Diese stellt den Druck in der Blutdruckmanschette 400 ein und übernimmt den gesamten weiteren Ablauf der Blutdruckmessung.

Die Druckmessung erfolgt mittels eines Drucksensors des Peritonealdialysegerätes. Die Anzeige des gemessenen Blutdruckes erfolgt an einem Display des Peritonealdialysegerätes. Die Speicherung und ggf. weitere Verarbeitung der Blutdruckwerte kann ebenfalls in dem Peritonealdialysegerät erfolgen.

Nach der Blutdruckmessung wird das Ventil 200 wieder in seine in der Figur gezeigte Stellung bewegt. In dieser Ventilstellung sind die Anschlüsse 2 und 3, nicht jedoch die Anschlüsse 2 und 1 verbunden; somit besteht keine Verbindung zwischen der Druckseite des Kompressors 100 und der Blutdruckmanschette 400. Die Rückstellung des Ventils 200 kann automatisch erfolgen oder durch Veranlassung durch den Nutzer.

Vorzugsweise ist das Ventil 200 z.B. durch eine Feder in seine in der Figur gezeigte Stellung vorgespannt, so dass es sich abgesehen von der Zeitspanne der Blutdruckmessung stets in der in der Figur dargestellten Position befindet. In dieser Position ist der Kompressor mit seiner Druckseite über die Leitung 310 mit dem Pneumatiksystem des Peritonealdialysegerätes verbunden.

## Patentansprüche

1. Dialysegerät, insbesondere Peritonealdialysegerät, mit
einem Kompressor,
einem pneumatischen Verbraucher, der mit dem Kompressor derart in Verbindung steht, dass dieser von dem Kompressor mit Druckluft beaufschlagbar ist, und
einer Blutdruckmanschette, die derart mit dem Dialysegerät in Verbindung steht oder verbindbar ist, dass die Blutdruckmanschette durch den Kompressor mit Druckluft versorgbar ist, wobei
ein Ventil zwischen dem Kompressor und der Blutdruckmanschette angeordnet ist, mittels dessen eine Leitung zwischen dem Kompressor und der Blutdruckmanschette absperrbar oder freigebbar ist,
**dadurch gekennzeichnet, dass**
das Ventil ein Mehrwegeventil ist, das dazu ausgelegt ist, entweder die Blutdruckmanschette oder sonstige Verbraucher des Dialysegeräts mit dem Kompressor in Druckluft-Verbindung zu bringen.

2. Dialysegerät nach Anspruch 1, **dadurch gekennzeichnet, dass** das Dialysegerät wenigstens einen Anschluss aufweist, mit dem die Blutdruckmanschette lösbar verbunden oder lösbar verbindbar ist.

3. Dialysegerät nach Anspruch 2, **dadurch gekennzeichnet, dass** es sich bei dem Anschluss um eine Steckverbindung handelt.

4. Dialysegerät nach Anspruch 1, **dadurch gekennzeichnet, dass** das Ventil als 2-Wege-Ventil ausgebildet ist.

5. Dialysegerät nach einem der vorhergehenden Ansprüche, **dadurch gekennzeichnet, dass** das Dialysegerät eine Steuerungs- oder Regelungseinheit aufweist, die derart ausgebildet ist, dass diese die Versorgung der Blutdruckmanschette mit Druckluft und/oder den Vorgang der Blutdruckmessung steuert oder regelt.

6. Dialysegerät nach Anspruch 5, **dadurch gekennzeichnet, dass** die Steuerungs- oder Regelungseinheit derart ausgebildet ist, dass diese den Betrieb des Dialysegerätes steuert oder regelt.

7. Dialysegerät nach einem der vorhergehenden Ansprüche, **dadurch gekennzeichnet, dass** wenigstens ein Drucksensor zur Erfassung des Blutdruckes vorhanden ist, wobei dieser Sensor einen Bestandteil des Dialysegerätes bildet oder mit dem Dialysegerät derart in Verbindung steht, dass der gemessene Druckwert an dem Dialysegerät anzeigbar ist.

8. Dialysegerät nach einem der vorhergehenden Ansprüche, **dadurch gekennzeichnet, dass** das Dialysegerät derart ausgebildet ist, dass die Datenerfassung, Speicherung und Verarbeitung der durch Einsatz der Blutdruckmanschette gemessenen Daten in dem Dialysegerät erfolgt.

9. Dialysegerät nach einem der vorhergehenden Ansprüche, **dadurch gekennzeichnet, dass** sämtliche zur Blutdruckmessung und Verarbeitung der gemessenen Daten erforderlichen Mittel außer der Blutdruckmanschette in dem Dialysegerät angeordnet sind.

## Claims

1. A dialyzer, in particular a peritoneal dialyzer, comprising
a compressor,
a pneumatic consumer, which is in communication with the compressor such that it can be acted on by compressed air from the compressor, and
a blood pressure cuff, which is in communication or can communicate with the dialyzer in such a way, that the blood pressure cuff can by supplied with compressed air by the compressor, wherein
a valve is arranged between the compressor and the blood pressure cuff, by means of which valve a line between the compressor and the blood pressure cuff can be blocked or unblocked,
**characterized in that**
the valve is a multiway valve, which is designed such as to bring either the blood pressure cuff or other consumers in compressed air communication with the compressor.

2. A dialyzer in accordance with claim 1, **characterized in that** the dialyzer has at least one connection to which the blood pressure cuff is or can be releasably connected.

3. A dialyzer in accordance with claim 2, **characterized in that** the connection is a plug connection.

4. A dialyzer according to claim 1, **characterized in that** the valve is configured as a 2-way-valve.

5. A dialyzer in accordance with one of the preceding claims, **characterized in that** the dialyzer has a control or regulation unit which is configured such that it controls or regulates the supply of the blood pressure cuff with compressed air and/or the procedure of the blood pressure measurement.

6. A dialyzer in accordance with claim 6, **characterized in that** the control or regulation unit is configured such that it controls or regulates the operation of the dialyzer.

7. A dialyzer in accordance with one of the preceding claims, **characterized in that** at least one pressure sensor is present for detecting the blood pressure, wherein this sensor forms an integral component of the dialyzer or is in communication with the dialyzer such that the measured pressure value can be displayed at the dialyzer.

8. A dialyzer in accordance with one of the preceding claims, **characterized in that** the dialyzer is configured such that the data acquisition, storage and processing of the data measured by use of the blood pressure cuff takes place in the dialyzer.

9. A dialyzer in accordance with one of the preceding claims, **characterized in that** all of the means required for the blood pressure measurement and for the processing of the measured data are arranged in the dialyzer, with the exception of the blood pressure cuff.

## Revendications

1. Appareil de dialyse, notamment appareil de dialyse péritonéale, comprenant
un compresseur,
un consommateur pneumatique qui est en liaison avec le compresseur de manière à ce que celui-ci puisse être alimenté en air comprimé par le compresseur, et
un brassard de tensiomètre qui est en liaison avec l'appareil de dialyse ou peut y être relié de manière à ce que le brassard de tensiomètre puisse être alimenté en air comprimé par le compresseur,
une valve étant disposée entre le compresseur et le brassard de tensiomètre, au moyen de laquelle une conduite peut être bloquée ou libérée entre le compresseur et le brassard de tensiomètre,
**caractérisé en ce que**
la valve est une vanne à plusieurs voies qui est conçue pour mettre en liaison d'air comprimé avec le compresseur soit le brassard de tensiomètre soit d'autres consommateurs de l'appareil de dialyse.

2. Appareil de dialyse selon la revendication 1, **caractérisé en ce que** l'appareil de dialyse présente au moins un raccord, auquel le brassard de tensiomètre est relié de manière amovible ou peut être raccordé de manière amovible.

3. Appareil de dialyse selon la revendication 2, **caractérisé en ce que** le raccord est une fiche de raccordement.

4. Appareil de dialyse selon la revendication 1, **caractérisé en ce que** la vanne est réalisée comme vanne à 2 voies.

5. Appareil de dialyse selon l'une quelconque des revendications précédentes, **caractérisé en ce que** l'appareil de dialyse présente une unité de commande et de réglage qui est réalisée de manière à ce que celle-ci commande ou règle l'alimentation du brassard de tensiomètre en air comprimé et/ou l'opération de mesure de la tension artérielle.

6. Appareil de dialyse selon la revendication 5, **caractérisé en ce que** l'unité de commande et de réglage est réalisée de manière à commander ou régler le fonctionnement de l'appareil de dialyse.

7. Appareil de dialyse selon l'une quelconque des revendications précédentes, **caractérisé en ce qu'**au moins un capteur de pression est présent pour détecter la tension artérielle, ce capteur formant une partie de l'appareil de dialyse ou étant en liaison avec l'appareil de dialyse de manière à ce que la valeur de tension mesurée puisse être affichée sur l'appareil de dialyse.

8. Appareil de dialyse selon l'une quelconque des revendications précédentes, **caractérisé en ce que** l'appareil de dialyse est réalisé de manière à ce que la saisie de données, la mémorisation et le traitement des données mesurées en raison de l'utilisation du brassard de tensiomètre aient lieu dans l'appareil de dialyse.

9. Appareil de dialyse selon l'une quelconque des revendications précédentes, **caractérisé en ce que** tous les moyens nécessaires pour la mesure de la tension artérielle et le traitement des données mesurées sont disposés dans l'appareil de dialyse, excepté le brassard de tensiomètre.
